# EUROPEAN PATENT SPECIFICATION

(11) **EP 4 208 134 B1**
(45) Date of publication and mention of the grant of the patent: **09.12.2026**
(21) Application number: 21769780.4
(22) Date of filing: 31.08.2021
(51) Int. Cl.: A61F 5/441, A61F 5/445

(54) **AN OSTOMY APPLIANCE**
EINE STOMAVORRICHTUNG
UN APPAREIL POUR STOMIE

(30) Priority: 03.09.2020 GB 202013883
(43) Date of publication of application: 12.07.2023
(73) Proprietor: Salts Healthcare Limited, Birmingham, West Midlands B7 4AA (GB)
(72) Inventor: ALFARO, Jesus, Birmingham West Midlands B7 4AA (GB); HOWARD, Lee, Birminghm West Midlands B7 4AA (GB)
(74) Representative: Forresters IP LLP
(86) International application number: PCT/GB2021/052244
(87) International publication number: WO 2022/049368

(56) References cited:
- EP-A1- 0 283 612
- WO-A1-2015/164832
- GB-A- 2 510 398
- GB-A- 2 534 012

## Description

### Background

Ostomy appliances are well known. Typically ostomy appliances have first and second walls which are connected at their peripheries to form a cavity for the collection of waste. The first or second wall is provided with a stoma-receiving opening for receipt of a user's stoma. The stoma-receiving opening is typically surrounded by a connection member such as a flange or an adhesive wafer (typically formed of hydrocolloid). A common issue with ostomy appliances is that of "pancaking". This can be caused by waste entering the cavity and adhering to both the first and second walls. This then adheres the first and second walls to each other and prevents further waste from readily entering the cavity and falling to a bottom thereof. This issue may also be generated by static charge or adhesive forces between the first and second walls, and/or the presence of a relative vacuum in the cavity, which result in the first and second walls adhering to each other and preventing waste from entering the cavity. In the most severe cases waste which cannot enter the cavity and fall to a bottom thereof may cause detachment of the connection member from a user. This is clearly undesirable and a source of embarrassment for a user.

Example prior art devices are shown in GB2510398, WO2015/164832 and GB2534012, which discloses the preamble of claim 1.

The present invention seeks to address these problems.

According to a first aspect of the invention we provide an ostomy appliance including:
first and second walls connected to each other at or near their peripheries by a peripheral connection;
a stoma-receiving opening for receipt of a stoma;
a collecting cavity defined between the first and second walls wherein the stoma-receiving opening is fluidly connected to the collecting cavity;
a connection member connected to the first wall for attaching the appliance to a user or for attaching the appliance to a flange for attaching the appliance to a user;
a gas vent in the second wall for venting gas from the collecting cavity to atmosphere;
a filter positioned over the gas vent, said filter defining a first discrete area;
a third wall including an outwardly facing surface which faces away from the first and second walls, said third wall optionally connected to the first and/or second wall at or near its periphery;
wherein the third wall overlies the filter;
wherein the second wall includes a first surface and the third wall includes a second surface;
wherein the first and second surfaces face each other and are connected at a connection;
wherein the connection defines a second discrete area; and
wherein movement of the third wall away from the first wall at least partially separates the first and second walls so as to permit air to flow through the gas vent into the collecting cavity.

The filter may define a first discrete area.

The connection may define a second discrete area.

The third wall may be or may include a comfort layer.

The ostomy appliance may include a plurality of connections between the second and third walls.

The connection or at least one of the connections between the second and third walls may be an adhesive connection.

The connection or at least one of the connections between second and third walls may be provided by a weld.

The connection or at least one of the connections may be positioned vertically below the filter, in an in use position of the appliance.

The connection or at least one of the connections may be positioned completely or at least partially above a generally horizontal line which extends through the stoma-receiving opening.

The connection or at least one of the connections may have a generally elongate axis which is inclined to a generally vertical axis of the ostomy appliance.

The connection or at least one of the connections may have a generally elongate axis which intersects the filter and/or gas vent.

The ostomy appliance may include first and second connections between the second and third walls. The first and second connections may be mirror images of each other.

The third wall may be positioned in an upper portion of the appliance. The third wall may be connected to the second wall of the appliance.

The third wall may cover a top portion of the appliance. The third wall may extend downwardly at least past a main horizontal axis of the stoma-receiving opening.

Embodiments of the invention will now be described by way of example only with reference to the accompanying drawings, of which:
Figure 1 is a front view of a first embodiment of an ostomy appliance in accordance with the present invention;
Figure 2 is a further front view of the ostomy appliance of figure 1;
Figure 3 is a rear view of the ostomy appliance of figure 1;
Figure 4 is a cross-sectional view of the ostomy appliance of figure 1 looking at the section of the ostomy appliance indicated by the line P;
Figure 5 is a further cross-sectional view of the ostomy appliance of figure 1 looking at the section of the ostomy appliance indicated by the line P;
Figure 6 is a further cross-sectional view of the ostomy appliance of figure 1 looking at the section of the ostomy appliance indicated by the line P with the section shown in dashed lines in figure 1 shown in solid lines;
Figure 7 is a top view of the ostomy appliance of figure 1 with the first, second and third walls adjacent each other in a partially open position;
Figure 8 is a front view of a second embodiment of an ostomy appliance in accordance with the present invention;
Figure 9 is a front view of an ostomy appliance not in accordance with the present invention; and
Figure 10 is a cross-sectional view of the ostomy appliance of figure 9 looking at the section of the ostomy appliance indicated by the ostomy appliance indicated by the line P.

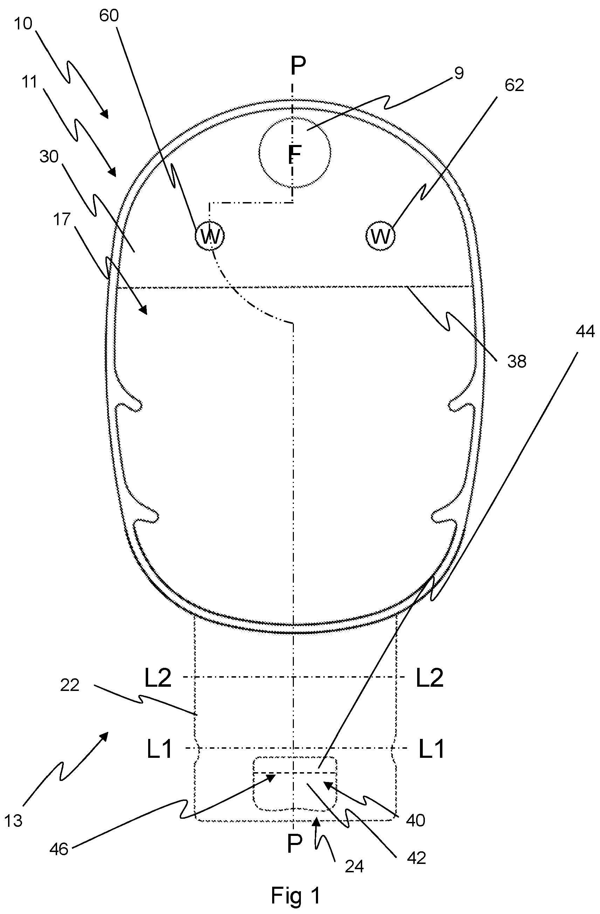

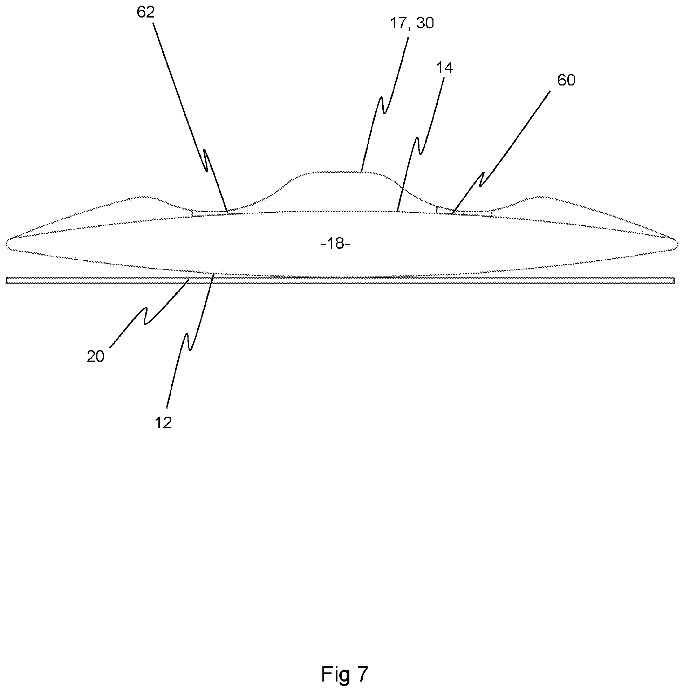

Referring to figures 1 to 7, these show a first embodiment of an ostomy appliance in accordance with the present invention, generally at 10. The ostomy appliance 10 has a top, or upper end, 11 and a bottom, or lower end, 13. The ostomy appliance 10 has first and second walls 12, 14 (preferably made from a plastics film material, which may be multi-layer) connected to each other at or near their peripheries, by any suitable means known in the art. For example, they could be adhered to each other or heat welded.

The first wall 12 includes a stoma-receiving opening 16 and supports a connection member or flange 20 for attaching the appliance 10 to a user. In the embodiment shown, the connection member 20 is a hydrocolloid wafer for securing the appliance 10 to the skin of a user around their stoma. Alternatively, the connection member 20 may be for attaching the appliance 10 to a flange for attaching the appliance to a user (e.g. a two-piece appliance as it is known in the art).

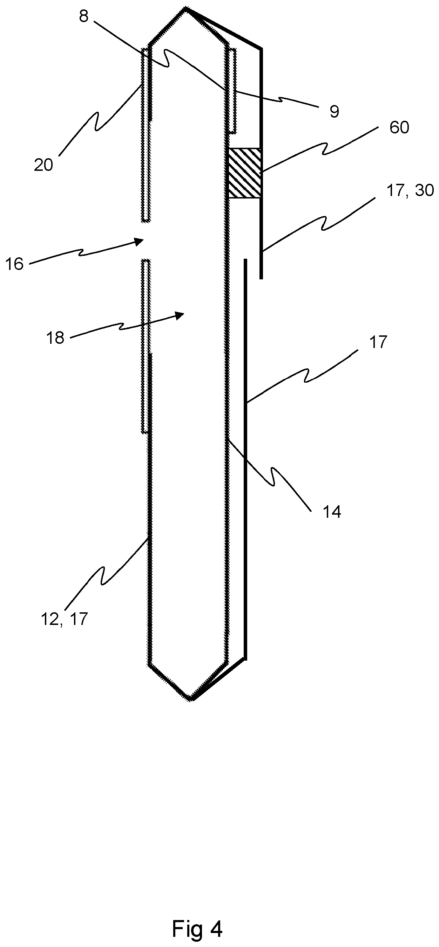

Referring to figure 4, the first and second walls 12, 14 define a waste collecting cavity 18 therebetween which is fluidly connected to the stoma-receiving opening 16. In the present embodiment the first and second walls 12, 14 include, first, respectively outwardly facing surfaces 32, 34. The first and second walls 12, 14 also include, second, respectively inwardly facing surfaces 52, 54. The first and second walls 12, 14, and in particular the outwardly facing surfaces 32, 34, are covered by comfort layers 17 though these are not essential. For example only the first or second wall 12, 14 may be provided with a comfort layer, or neither the first nor the second wall 12, 14 may be provided with a comfort layer. The comfort layers 17 may be laminated to the first and/or second walls 12, 14.

The appliance 10 includes a filter 9 positioned over a suitable gas vent 8 in the second 14 wall, as is well known in the art. In particular, the filter 9 is positioned on the first, outwardly facing, surface 34 of the second wall 44 (though in other embodiments the filter 9 may be positioned on the second, inwardly facing, surface 34 of the second wall 14). In other embodiments the filter 9 may be positioned over a suitable gas vent in the first wall 12 (on either the first or second surfaces 32, 52 of the first wall 12). In further envisaged embodiments a plurality of gas vents 8 may be provided in the first and/or second walls 12, 14. Filters 9 may be positioned over the respective gas vents 8 in the first and/or second walls 12, 14 (on the first and/or second respective surfaces 32, 34, 52, 54). The filter 9, or each filter in those embodiments where there are a plurality of filters, defines a first discrete area F.

A further third wall 30 at least partially overlies the second wall 14. The third wall 30 may also overlie the or each filter 9 but this need not necessarily be the case. The third wall 30 is optionally connected to the first and/or second walls 12, 14. In particular the third wall 30 may be connected to the first and/or second walls 12, 14 at or near its peripheries except for a bottom edge 38 thereof. In the present embodiment the third wall 30 is positioned in an upper portion 11 of the ostomy appliance 10 and is connected to the second wall 14 of the ostomy appliance 10. The third wall 30 covers a top portion of the appliance 10 and extends downwardly at least past a main horizontal axis of the stoma-receiving opening 16. The third wall 30 may be made from a plastics film material, which may be multi-layer. Alternatively the third wall 30 may be made from other materials. For example, in the present embodiment the third wall 30 is formed by a portion of the comfort layer 17 which covers the second wall 14. The third wall 30 in the present embodiment also includes a plastics film material which the comfort layer 17 overlies at least partially. In particular a first, outwardly facing, surface 36 of the third wall 30 is attached to a portion of the comfort layer 17 by a lamination process.

As explained above the third wall 30 has a first, outwardly facing, surface 36. The first, outwardly facing, surface 36 faces away from a user and the first and second walls 12, 14. The third wall 30 also includes a second, inwardly facing, surface 56 which faces towards the user and the first and second walls 12, 14. In particular, the second surface 56 of the third wall 30 and the first surface 34 of the second wall 14 face each other and are connected at a connection 60. The connection 60 defines a second discrete area W. The connection 60 is described in more detail later.

The third wall 30 may cover a third area T of the ostomy appliance 10. The first and second areas F and W may lie within the third area T. In other embodiments only the second area W may lie within the third area T.

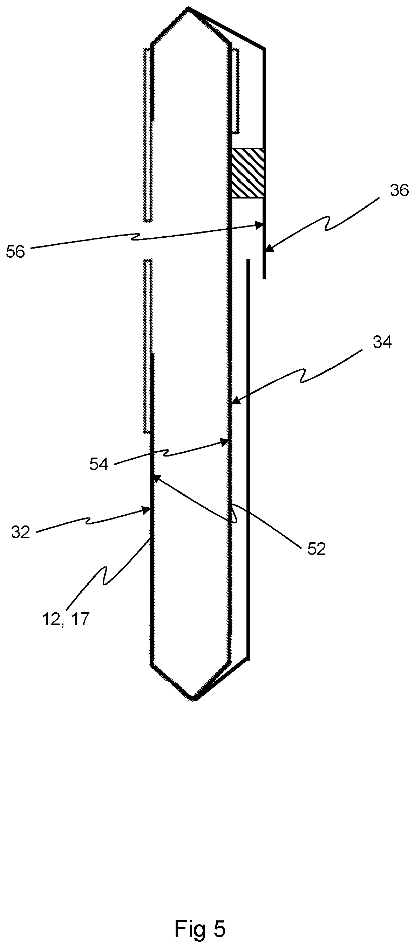

The third wall 30 is movable in a direction away from the first wall 12. The third wall 30 is also movable in a direction away from the second wall 14. The third wall 30 is also movable in a direction generally away from a user. Movement of the third wall 30 away from the first wall 12 at least partially separates the first and second walls 12, 14, as shown at figure 5. In particular movement of the third wall 30 causes movement of the connection 60 away from the first wall 12. This in turn causes movement of the second wall 14 away from the first wall 12. Movement of the third wall 30 in this manner permits air to flow through the gas vent 8 into the collecting cavity 18.

Such a construction is advantageous. By moving the third wall as described above a user is able to solve the issue of pancaking by overcoming the adhesive force caused by waste sticking the first and second walls together. Such movement of the third wall also assists in overcoming pancaking which occurs due to vacuum/static forces between the first and second walls 12, 14. By moving the third wall 30 the second wall 14 is moved away from the first wall 12 so as to overcome any static forces between the first and second walls 12, 14. In other words, movement of the third wall away from the first wall 12 increases the volume of the waste collecting cavity 18 which creates a greater vacuum, or pressure differential between the waste collecting cavity 18, a user and atmosphere. To resolve the pressure difference either air will enter the ostomy appliance 10 through the gas vent 8 and thus assist in maintaining the first and second walls 12, 14 in a spaced apart configuration or waste will enter through the stoma-receiving opening 16 and enter the waste collecting cavity 18. This waste will then maintain the first and second walls 12, 14 in a spaced apart configuration. Therefore, the invention enables a user to easily remove the issue of pancaking and continue to use the ostomy appliance without the need to replace the ostomy appliance.

In the present embodiment first and second connections 60, 62 are provided between the second and third walls 14, 30. However, in other embodiments only a single connection 60 may be provided. In further envisaged embodiments a plurality of connections 60 may be provided between the second and third walls 14, 30. The connections 60, 62 are generally the same shape. In other embodiments the connections 60 may be different shapes.

In the present embodiment the first and second connections 60, 62 are generally circular. However, the first and second connections 60, 62 may be other shapes without departing from the scope of the present invention. For example, the connections 60 may be elliptical, obround/racetrack shaped, triangular or any other polygonal shape without departing from the scope of the present invention.

The or each connection 60, 62 may be provided by an adhesive connection between the second and third walls 14, 30. In addition the or each connection 60, 62 may be provided by a weld between the second and third walls 14, 30.

The connection, or at least one of the connections, 60, 62 is positioned vertically below the filter 9, in an in use position of the ostomy appliance 10. By vertically we mean at a position below the filter 9 on a midline M which extends generally between the top 11 and bottom 13 of the appliance 10 which is generally perpendicular to a horizontal line H which extends through the stoma-receiving opening 16 and a main axis of the stoma-receiving opening 16 through which a stoma extends in use. In other words, we mean that the connections 60, 62 are not necessarily directly beneath the filter 9, but are positioned vertically closer to the bottom 13 of the ostomy appliance 10 than the filter 9. In some embodiments the or each connection 60 may be provided directly below the filter 9. The connection, or at least one of the connections 60, 62 may be positioned vertically above the stoma-receiving opening 16. In particular, the connection, or at least one of the connections, 60, 62 is positioned completely or at least partially above the generally horizontal line H which extends through the stoma-receiving opening 16. In embodiments, the connections 60, 62 may be positioned at a periphery of a maximum size to which the stoma-receiving opening 16 may be enlarged.

The first and second connections 60, 62 lie either side of the filter 9 generally opposite each other. In particular, the first and second connections 60, 62 are mirror images of each other.

In the present embodiment the first and second connections 60, 62 are separate from the peripheral connection between the first, second and third walls 12, 14, 30. However, in other embodiments the connection(s) 60, 62 may be connected to a periphery of the ostomy appliance. In particular, in those embodiments where the connection(s) 60, 62 are provided as welds the connection(s) 60, 62 may be formed as part of a peripheral weld between the first, second and third walls 12, 14, 30.

The connection(s) 60, 62 may extend inwardly from a periphery of the ostomy appliance 10. Such a construction may be advantageous in guiding a user's fingers to the most effective place at which to pull the third wall 30 away from the first wall 12.

The connection(s) 60, 62 may be inclined with respect to the midline M described above. Again, this construction may be advantageous in guiding a user's fingers to the most effective place at which to pull the third wall 30 away from the first wall 12.

The connection(s) 60, 62 may, in hand with the second and third walls 14, 30, define a pocket for receipt of a user's fingers. The pocket may also be defined at least partly by the filter 9. In particular the pocket may be defined between the second and third walls 14, 30, the first and second connections 60, 62 and the filter 9.

In use a user may use the pocket by moving the bottom edge 38 of the third wall 30 away from the second wall 14 and extending their fingers into the pocket. In the present embodiment this would be between the first and second connections 60, 62. A user may then move their fingers away from the first wall 12 and thus move the third wall 30 in a direction away from the first wall 12 as described above.

In the present embodiment the second discrete area W of each connection 60, 62 is less than the first discrete area F of the filter 9. In particular the combined area W+W defined by the connections 60, 62 is less than the area F defined by the filter 9. However, in other embodiments the area W defined by the or each connection 60, 62 may be the same as the area F defined by the filter 9 or may be greater than the area F defined by the filter 9, either separately or in combination.

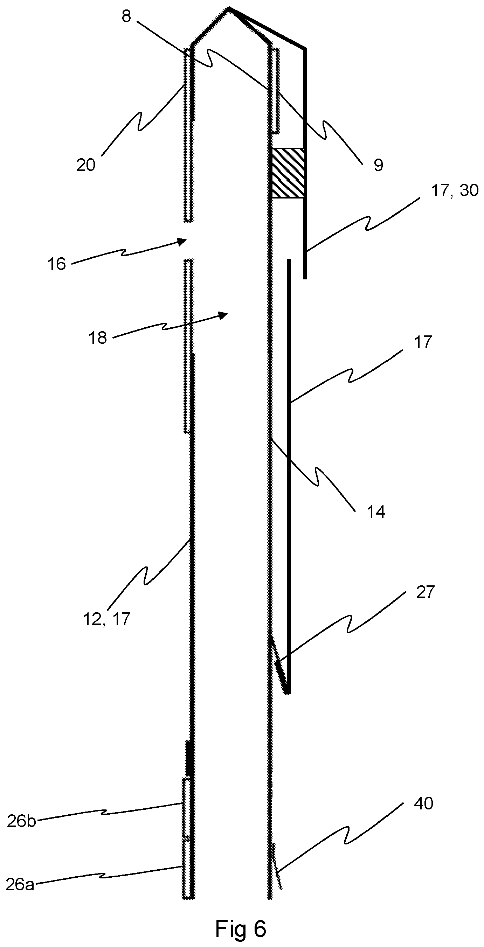

The lower end 13 of the appliance 10 may be provided with an outlet 22 including an outlet wall (as shown in dashed lines in figures 1 to 3 and as shown in figure 6). The outlet 22 may be formed by portions of the first and second walls 12, 14, again, as is well known in the art. In other words the outlet 22 may be a continuation of one or both of the first and second walls 12, 14 which define the waste collecting cavity 18. In other embodiments the outlet 22 may be formed from a wall which is separate to the first and second walls 12, 14. Such an outlet 22 may be connected to one of the first and/or second walls which define the waste collecting cavity 18.

The outlet 22 may extend downwardly away from the stoma-receiving opening 16 and terminate at an opening 24. The opening 24 may permit waste collected in the collecting cavity 18 to be expelled from the appliance 10. The opening 24 may be movable between a first, closed, condition and a second, open, condition. When the opening 24 is in the second, open, condition waste may be permitted to move from the waste collecting cavity 18 through the outlet 22 and out of the opening 24 to exit the ostomy appliance 10.

To assist a user in moving the outlet opening from the first condition to the second condition the ostomy appliance 10 may include a user graspable member, or tab, 40 which is positioned at or near the outlet opening 24 and is attached to the outlet wall 22 by an attachment portion 44. The attachment portion 44 may be adhered or welded to the outlet wall 22.

The user graspable member 40 may also include a graspable portion 42 which is connected to the attachment portion 44 by a connection portion 46. A user may operate the user graspable member 40 by grasping the graspable portion 42 and moving the graspable portion 42 in a direction away from the outlet 22 and a user.

As shown in the figures, first and second elongate stiffening members 26a, 26b may be provided to assist with opening, closing and sealing of the opening 24. However, in other embodiments only a single stiffening member 26a may be provided. The stiffening member 26a may be positioned at or near the opening 24.

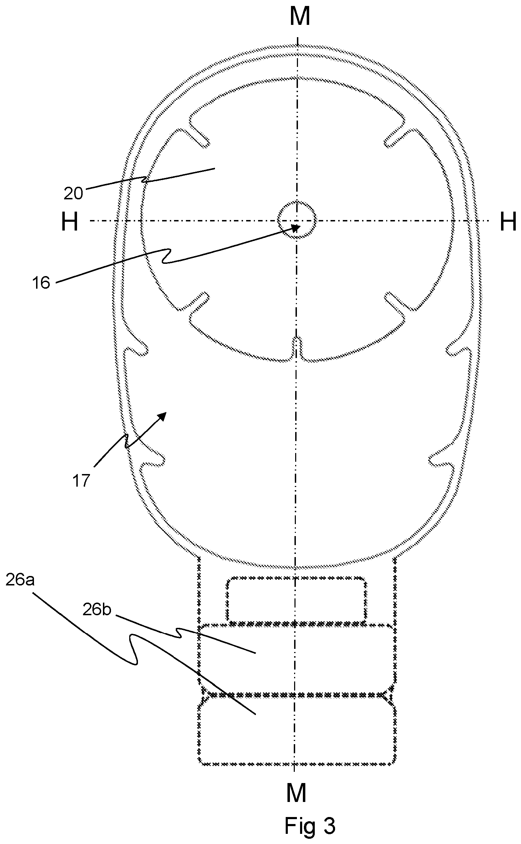

The first stiffening member 26a may be connected, e.g. adhered or welded, to the exterior surface of the first wall 12, which faces the user and is positioned at or near the outlet opening 24. In alternative embodiments the first stiffening member 26a forms part of the outlet 22. The first stiffening member 26a may extend laterally across the outlet 22 such that it extends generally perpendicularly to the direction in which waste exits the appliance 10, as shown in figure 3.

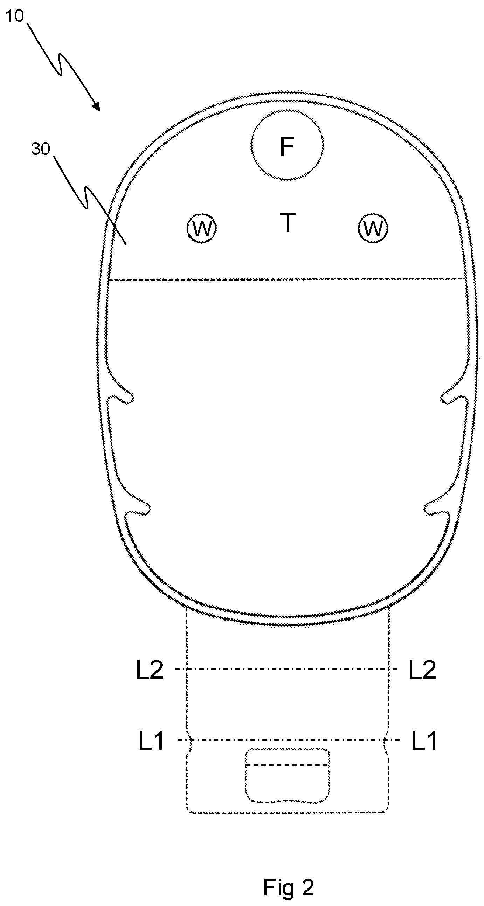

The second stiffening member 26b may be positioned adjacent and above the first stiffening member 26a, further away from the opening 24 of the outlet 22 than the first stiffening member 26a, and is connected, e.g. adhered or welded, to the exterior surface of the first wall 12 (the same wall as the first stiffening member 26a). The second stiffening member 26b may also extend laterally across the outlet 22 such that it extends generally perpendicularly to the direction in which waste exits the appliance 10, as shown in figure 2. The stiffening members 26a, 26b may be substantially the same.

The outlet 22, when present, may be foldable, or rollable, between an extended position and a stowed position. The ostomy appliance 10 may include a security flap 27 (which may lie underneath the comfort layer 17) to secure the outlet in its stowed position. In the stowed position the attachment portion 44 and graspable portion 42 may lie in generally the same plane.

The outlet 22 may be stored, or stowed, by being rolled up around the stiffening members 26a, 26b, as is generally known in the art. The stiffening members 26a, 26b may define fold lines L1, L2 which the outlet 22 folds about.

The stiffening members 26a, 26b may in particular be rolled up two or three times, to seal the opening 24 in a closed condition. Any suitable fastening means, e.g. hook, and loop (Velcro) or adhesive, may be provided on respective faces of the flap 27 and outlet 22 to secure the outlet 22.

A second embodiment of the invention will now be described. Features shared in common with the first embodiment of the invention will be provided with the same reference numerals with the addition of 100. Only the differences between the first and second embodiments will be described.

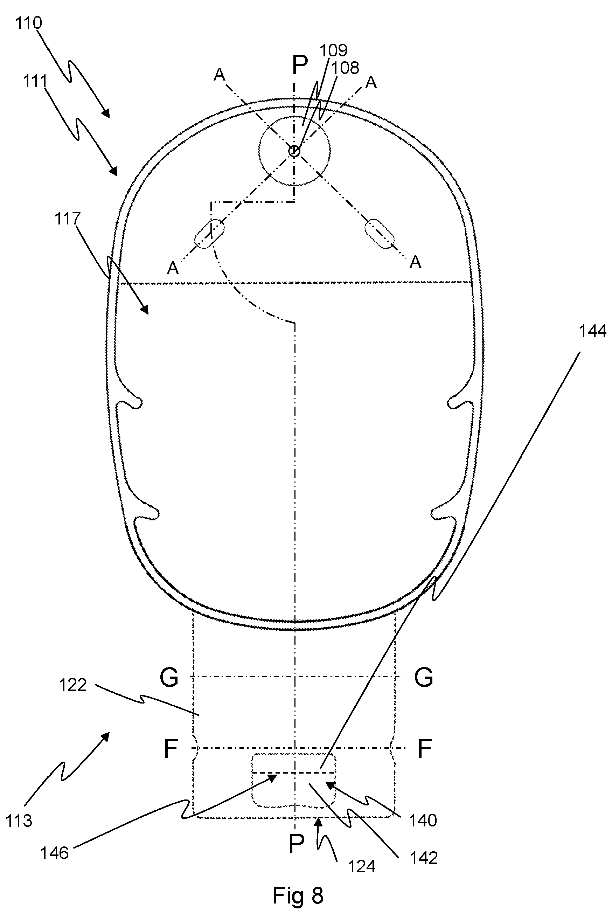

Figure 8 shows a second embodiment of the invention. In this embodiment of the invention the connections 160, 162 between the second and third walls 14, 30 are provided as obrounds of adhesive. By this we mean that each connection 160, 162 has first and second generally parallel sides 170, 172 which are connected by third and fourth generally curved sides 174, 176. The third and fourth sides 174, 176, in particular, are semi-circular.

The first and second sides 170, 172 are inclined to the midline M and/or a generally vertical axis of the ostomy appliance. In particular the connection(s) 160, 162 each have a generally elongate axis A, with which the first and second sides 170, 172 may be parallel, which is inclined to a generally vertical axis of the ostomy appliance 110. The generally elongate axis A and/or the first and second sides 170, 172 may be inclined to the midline M at an angle of roughly 45 degrees. However, other angles may be used without departing from the scope of the invention. The elongate axis A of the or each connection 160, 162 intersects the gas vent 8 and the filter 9. In other embodiments the elongate axes A of the connections 160, 162 intersect each other below the gas vent 8 and/or filter 9. In further envisaged embodiments the elongate axes A of the connections 160, 162 may intersect each other above the gas vent 8 and/or filter 9. As in the first embodiment the first and second connections 160, 162 are between the second and third walls, 114, 130 and are mirror images of each other.

An ostomy appliance, not belonging to the invention, will now be described. Features shared in common with the first embodiment of the invention will be provided with the same reference numerals with the addition of 200. Only the differences between the first and second embodiments will be described.

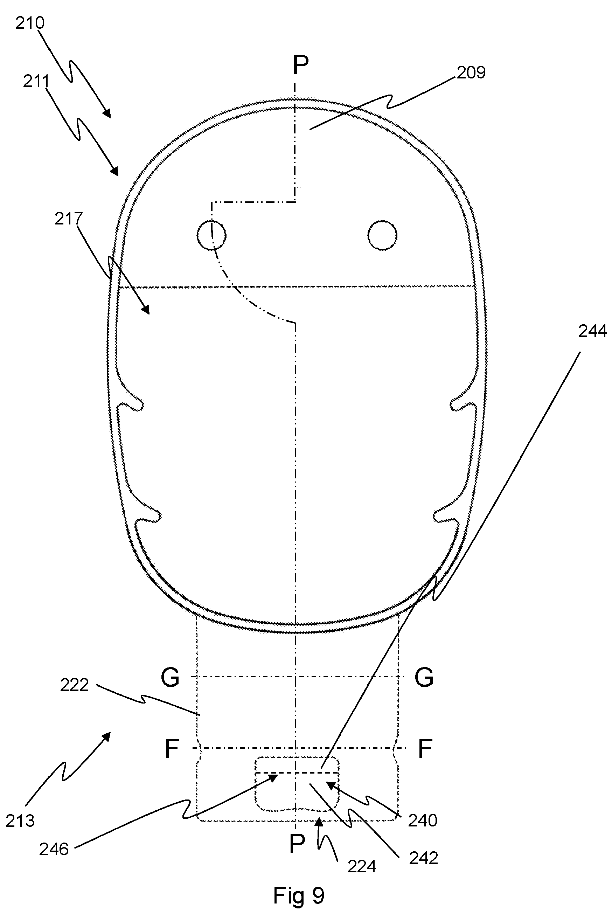

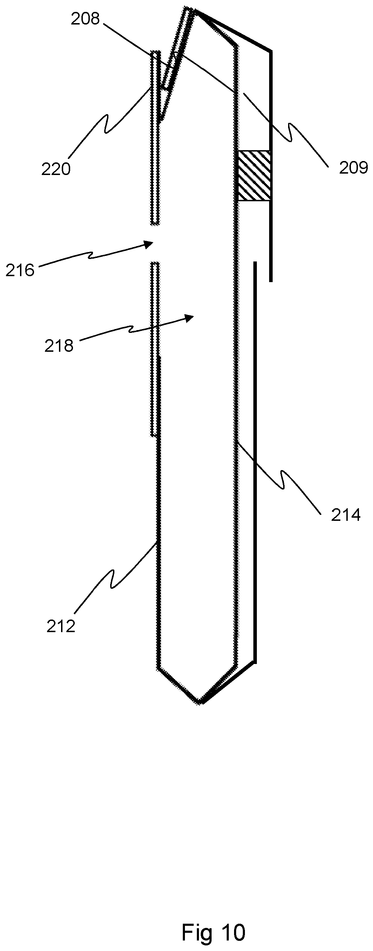

Figures 9 and 10 show a an ostomy appliance, not falling within the scope of claim 1, wherein the gas vent 208 is provided in the first wall 212 for venting gas from the collecting cavity 218 to atmosphere. The filter 209 is positioned over the gas vent 208 on an outwardly facing surface 252 of the first wall 212. In other embodiments the filter 209 may be positioned over the gas vent 208 on an inwardly facing surface 232 of the first wall 212.

Operation of the ostomy appliance 210 is similar to the ostomy appliance 10 and 110 and so will not be described here again.

Representative features are set out in the following clauses, which stand alone or may be combined, in any combination, with one or more features disclosed in the text and/or drawings of the specification.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

Although certain example embodiments of the invention have been described, the scope of the appended claims is not intended to be limited solely to these embodiments.

## Claims

1. An ostomy appliance (10, 110) including:
first and second walls (12, 14, 112, 114) connected to each other at or near their peripheries by a peripheral connection;
a stoma-receiving opening (16, 116) for receipt of a stoma;
a collecting cavity (18, 118) defined between the first and second walls wherein the stoma-receiving opening is fluidly connected to the collecting cavity;
a connection member (20, 120) connected to the first wall for attaching the appliance to a user or for attaching the appliance to a flange for attaching the appliance to a user;
a gas vent (8, 108) in the second wall for venting gas from the collecting cavity to atmosphere;
a filter (9, 109) positioned over the gas vent, said filter defining a first discrete area (F);
a third wall (30, 130) including an outwardly facing surface which faces away from the first and second walls, said third wall optionally connected to the first and/or second wall at or near its periphery;
wherein the third wall (30, 130) overlies the filter (9, 109);
wherein the second wall includes a first surface (34, 134) and the third wall includes a second surface (56, 156);
**characterized in that**
the first and second surfaces face each other and are connected at a connection (60, 62, 160, 162);
wherein the connection defines a second discrete area (W); and
wherein movement of the third wall (30, 130) away from the first wall (12, 112) at least partially separates the first and second walls (12, 14, 112, 114) so as to permit air to flow through the gas vent (8, 108) into the collecting cavity (18, 118).

2. An ostomy appliance (10, 110, 210) according to claim 1 wherein the third wall (30, 130, 230) is or includes a comfort layer.

3. An ostomy appliance (10, 110, 210) according to any preceding claim including a plurality of connections (60, 62, 160, 162, 260, 262) between the second and third walls (14, 30, 114, 130, 214, 230).

4. An ostomy appliance (10, 110, 210) according to any preceding claim wherein the connection (60, 62, 160, 162, 260, 262) or at least one of the connections between the second and third walls (14, 30, 114, 130, 214, 230) is an adhesive connection; and / or
wherein the connection or at least one of the connections between second and third walls is provided by a weld.

5. An ostomy appliance (10, 110, 210) according to any preceding claim wherein the connection (60, 62, 160, 162, 260, 262) or at least one of the connections is positioned vertically below the filter (9, 109, 209), in an in use position of the appliance.

6. An ostomy appliance (10, 110, 210) according to any preceding claim wherein the connection (60, 62, 160, 162, 260, 262) or at least one of the connections is positioned completely or at least partially above a generally horizontal line which extends through the stoma-receiving opening (16, 116, 216).

7. An ostomy appliance (110) according to any preceding claim wherein the connection (160, 162) or at least one of the connections has a generally elongate axis (A) which is inclined to a generally vertical axis of the ostomy appliance.

8. An ostomy appliance (110) according to any preceding claim wherein the connection (160, 162) or at least one of the connections has a generally elongate axis (A) which intersects the filter (9) and/or gas vent (8).

9. An ostomy appliance (10, 110, 210) according to any preceding claim when dependent, directly or indirectly, on claim 3 including first and second connections (60, 62, 160, 162, 260, 262) between the second and third walls (14, 30, 114, 130, 214, 230) where the first and second connections are mirror images of each other.

10. An ostomy appliance (10, 110, 210) according to any preceding claim wherein the third wall (30, 130, 230) is positioned in an upper portion of the appliance and is connected to the second wall (14, 114, 214) of the appliance.

11. An ostomy appliance (10, 110, 210) according to any preceding claim wherein the third wall (30, 130, 230) covers a top portion of the appliance and extends downwardly at least past a main horizontal axis of the stoma-receiving opening (16, 116, 216).

## Patentansprüche

1. Ostomievorrichtung (10, 110), die Folgendes aufweist:
eine erste und eine zweite Wand (12, 14, 112, 114), die an oder nahe ihren Peripherien durch eine Peripherieverbindung miteinander verbunden sind;
eine Stomaaufnahmeöffnung (16, 116) zur Aufnahme eines Stomas;
einen zwischen der ersten und der zweiten Wand gebildeten Auffangraum (18, 118), wobei die Stomaaufnahmeöffnung mit dem Auffangraum fluidisch verbunden ist;
ein mit der ersten Wand verbundenes Verbindungselement (20, 120) zum Befestigen der Vorrichtung an einem Benutzer oder zum Befestigen der Vorrichtung an einem Flansch zum Befestigen der Vorrichtung an einem Benutzer;
eine Gasentlüftung (8, 108) in der zweiten Wand zum Ablassen von Gas aus dem Auffangraum in die Atmosphäre;
einen über der Gasentlüftung positionierten Filter (9, 109), wobei der genannte Filter einen ersten diskreten Bereich (F) bildet;
eine dritte Wand (30, 130) mit einer nach außen weisenden Fläche, die von der ersten und der zweiten Wand abgewandt ist, wobei die genannte dritte Wand optional an oder nahe ihrer Peripherie mit der ersten und/oder der zweiten Wand verbunden ist;
wobei die dritte Wand (30, 130) den Filter (9, 109) überlagert;
wobei die zweite Wand eine erste Fläche (34, 134) und die dritte Wand eine zweite Fläche (56, 156) aufweist;
**dadurch gekennzeichnet, dass** die erste und die zweite Fläche einander zugewandt und an einer Verbindung (60, 62, 160, 162) miteinander verbunden sind;
wobei die Verbindung einen zweiten diskreten Bereich (W) definiert; und
wobei eine Bewegung der dritten Wand (30, 130) weg von der ersten Wand (12, 112) die erste und die zweite Wand (12, 14, 112, 114) zumindest teilweise voneinander trennt, um einen Luftstrom durch die Gasentlüftung (8, 108) in den Auffangraum (18, 118) zuzulassen.

2. Ostomievorrichtung (10, 110, 210) nach Anspruch 1, wobei die dritte Wand (30, 130, 230) eine Komfortschicht ist oder aufweist.

3. Ostomievorrichtung (10, 110, 210) nach einem vorherigen Anspruch mit mehreren Verbindungen (60, 62, 160, 162, 260, 262) zwischen der zweiten und der dritten Wand (14, 30, 114, 130, 214, 230).

4. Ostomievorrichtung (10, 110, 210) nach einem vorherigen Anspruch, wobei die Verbindung (60, 62, 160, 162, 260, 262) oder mindestens eine der Verbindungen zwischen der zweiten und der dritten Wand (14, 30, 114, 130, 214, 230) eine Klebeverbindung ist; und/oder
wobei die Verbindung oder mindestens eine der Verbindungen zwischen der zweiten und der dritten Wand durch eine Schweißung gebildet wird.

5. Ostomievorrichtung (10, 110, 210) nach einem vorherigen Anspruch, wobei die Verbindung (60, 62, 160, 162, 260, 262) oder mindestens eine der Verbindungen in einer Gebrauchsposition der Vorrichtung vertikal unterhalb des Filters (9, 109, 209) positioniert ist.

6. Ostomievorrichtung (10, 110, 210) nach einem vorherigen Anspruch, wobei die Verbindung (60, 62, 160, 162, 260, 262) oder mindestens eine der Verbindungen vollständig oder zumindest teilweise oberhalb einer allgemein horizontalen Linie positioniert ist, die sich durch die Stomaaufnahmeöffnung (16, 116, 216) erstreckt.

7. Ostomievorrichtung (110) nach einem vorherigen Anspruch, wobei die Verbindung (160, 162) oder mindestens eine der Verbindungen eine allgemein längliche Achse (A) hat, die gegenüber einer allgemein vertikalen Achse der Ostomievorrichtung geneigt ist.

8. Ostomievorrichtung (110) nach einem vorherigen Anspruch, wobei die Verbindung (160, 162) oder mindestens eine der Verbindungen eine allgemein längliche Achse (A) aufweist, die den Filter (9) und/oder die Gasentlüftung (8) schneidet.

9. Ostomievorrichtung (10, 110, 210) nach einem vorherigen Anspruch wenn direkt oder indirekt abhängig von Anspruch 3, mit einer ersten und einer zweiten Verbindung (60, 62, 160, 162, 260, 262) zwischen der zweiten und der dritten Wand (14, 30, 114, 130, 214, 230), wobei die erste und die zweite Verbindung spiegelbildlich zueinander sind.

10. Ostomievorrichtung (10, 110, 210) nach einem vorherigen Anspruch, wobei die dritte Wand (30, 130, 230) in einem oberen Abschnitt der Vorrichtung positioniert und mit der zweiten Wand (14, 114, 214) der Vorrichtung verbunden ist.

11. Ostomievorrichtung (10, 110, 210) nach einem vorherigen Anspruch, wobei die dritte Wand (30, 130, 230) einen oberen Abschnitt der Vorrichtung abdeckt und sich nach unten zumindest über eine horizontale Hauptachse der Stomaaufnahmeöffnung (16, 116, 216) hinaus erstreckt.

## Revendications

1. Appareillage stomique (10, 110), comprenant :
des première et deuxième parois (12, 14, 112, 114) reliées l'une à l'autre au niveau ou à proximité de leurs périphéries par une liaison périphérique ;
une ouverture de réception de stomie (16, 116) pour la réception d'une stomie ;
une cavité de collecte (18, 118) définie entre les première et deuxième parois, l'ouverture de réception de stomie étant reliée fluidiquement à la cavité de collecte ;
un élément de liaison (20, 120) relié à la première paroi pour fixer l'appareillage à un utilisateur ou pour fixer l'appareillage à une collerette pour fixer l'appareillage à un utilisateur ;
un évent de gaz (8, 108) dans la deuxième paroi pour évacuer du gaz depuis la cavité de collecte vers l'atmosphère ;
un filtre (9, 109) positionné sur l'évent de gaz, ledit filtre définissant une première zone distincte (F) ;
une troisième paroi (30, 130) comprenant une surface orientée vers l'extérieur qui est orientée à l'opposé des première et deuxième parois, ladite troisième paroi étant éventuellement reliée à la première et/ou à la deuxième paroi au niveau ou à proximité de sa périphérie ;
la troisième paroi (30, 130) recouvrant le filtre (9, 109) ;
la deuxième paroi comprenant une première surface (34, 134) et la troisième paroi comprenant une seconde surface (56, 156) ;
l'appareillage stomique étant **caractérisé en ce que** les première et seconde surfaces se font face et sont reliées au niveau d'une liaison (60, 62, 160, 162) ;
la liaison définissant une seconde zone distincte (W) ; et
un déplacement de la troisième paroi (30, 130) à l'écart de la première paroi (12, 112) séparant au moins en partie les première et deuxième parois (12, 14, 112, 114) de manière à permettre à de l'air de s'écouler par l'évent de gaz (8, 108) dans la cavité de collecte (18, 118).

2. Appareillage stomique (10, 110, 210) selon la revendication 1, la troisième paroi (30, 130, 230) étant ou comprenant une couche de confort.

3. Appareillage stomique (10, 110, 210) selon l'une quelconque des revendications précédentes, comprenant une pluralité de liaisons (60, 62, 160, 162, 260, 262) entre les deuxième et troisième parois (14, 30, 114, 130, 214, 230).

4. Appareillage stomique (10, 110, 210) selon l'une quelconque des revendications précédentes, la liaison (60, 62, 160, 162, 260, 262) ou au moins l'une des liaisons entre les deuxième et troisième parois (14, 30, 114, 130, 214, 230) étant une liaison adhésive ; et/ou
la liaison ou au moins l'une des liaisons entre les deuxième et troisième parois étant assurée par une soudure.

5. Appareillage stomique (10, 110, 210) selon l'une quelconque des revendications précédentes, la liaison (60, 62, 160, 162, 260, 262) ou au moins l'une des liaisons étant positionnée verticalement au-dessous du filtre (9, 109, 209), dans une position d'utilisation de l'appareillage.

6. Appareillage stomique (10, 110, 210) selon l'une quelconque des revendications précédentes, la liaison (60, 62, 160, 162, 260, 262) ou au moins l'une des liaisons étant positionnée complètement ou au moins en partie au-dessus d'une ligne généralement horizontale qui s'étend par l'ouverture de réception de stomie (16, 116, 216).

7. Appareillage stomique (110) selon l'une quelconque des revendications précédentes, la liaison (160, 162) ou au moins l'une des liaisons présentant un axe généralement allongé (A) qui est incliné par rapport à un axe généralement vertical de l'appareillage stomique.

8. Appareillage stomique (110) selon l'une quelconque des revendications précédentes, la liaison (160, 162) ou au moins l'une des liaisons présentant un axe généralement allongé (A) qui coupe le filtre (9) et/ou l'évent de gaz (8).

9. Appareillage stomique (10, 110, 210) selon l'une quelconque des revendications précédentes, lorsqu'elle dépend, directement ou indirectement, de la revendication 3, comprenant des première et seconde liaisons (60, 62, 160, 162, 260, 262) entre les deuxième et troisième parois (14, 30, 114, 130, 214, 230), les première et seconde liaisons étant des images symétriques l'une de l'autre.

10. Appareillage stomique (10, 110, 210) selon l'une quelconque des revendications précédentes, la troisième paroi (30, 130, 230) étant positionnée dans une partie supérieure de l'appareillage et étant reliée à la deuxième paroi (14, 114, 214) de l'appareillage.

11. Appareillage stomique (10, 110, 210) selon l'une quelconque des revendications précédentes, la troisième paroi (30, 130, 230) recouvrant une partie supérieure de l'appareillage et s'étendant vers le bas au moins au-delà d'un axe horizontal principal de l'ouverture de réception de stomie (16, 116, 216).
